# EUROPEAN PATENT APPLICATION

(11) **EP 4 282 400 A1**
(43) Date of publication of application: **29.11.2023**
(21) Application number: 21920188.6
(22) Date of filing: 19.01.2021
(51) Int. Cl.: A61K 9/08, A61K 38/00, C07K 14/00, A61P 31/04

(54) **ANTIMICROBIAL PEPTIDE LIQUID COMPOSITION AND FORMULATION THEREOF**

(71) Applicant: Jiangsu Protelight Pharmaceutical & Biotechnology Co., Ltd., Jiangyin, Jiangsu 214429 (CN)
(72) Inventor: CHEN, Yuxin, wuxi, Jiangsu 214429 (CN); CHEN, Mingxia, wuxi, Jiangsu 214429 (CN); CHEN, Wenxue, wuxi, Jiangsu 214429 (CN); HUANG, Xiaoxue, wuxi, Jiangsu 214429 (CN)
(74) Representative: CMS Cameron McKenna Nabarro Olswang LLP
(86) International application number: PCT/CN2021/072665
(87) International publication number: WO 2022/155783

(57) **Abstract**

An antimicrobial peptide composition comprising an antimicrobial peptide, at least one stabilizer, and a buffer system, the mass concentration of the antimicrobial peptide in the composition being 0.1%o to 10%o; and the buffer system being a phosphate buffer system or an acetate buffer system; the amino acid sequence of the antimicrobial peptide being: KWKSFLKTFaAbKTVLHTALKAISS. The antimicrobial peptide composition is a broad-spectrum anti-infective drug suitable for various primary skin infections caused by pathogenic bacteria, especially drug-resistant bacteria, and secondary skin infections such as an eczema co-infection and an ulcer co-infection, including persistent infectious diseases such as diabetic foot, burn wound infections, and decubitus ulcer infections, and the composition has broad application prospects.

## Description

### TECHNICAL FIELD

The present invention relates to the field of biological technology, and relates in particular to an antimicrobial peptide liquid composition and a preparation thereof.

### BACKGROUND

Antimicrobial drugs mainly include antibiotics and synthetic antibacterial drugs. Since the 1920s and 1930s, the discovery and application of antibiotics has saved countless lives. Antibiotics are substances that are produced by bacteria or other microorganisms during the course of the lives thereof and have the effect of inhibiting or killing pathogenic microorganisms, such as bacteria, spirochetes, mycoplasma, and chlamydia. Antibiotics that are already on the market mainly include β-lactams, macrolides, polysaccharides (vancomycin, teicoplanin), aminoglycosides, tetracyclines, chloramphenicols, peptide lactones (daptomycin), and the like.

If earlier humans benefited from antibiotics, then the world today must be committed to resolving the consequences of antibiotic abuse - the emergence and spread of drug-resistant bacteria. Super bacteria have developed insensitivity towards antibiotics, and "super super bacteria" exist in addition to "super bacteria". With the discovery of each and every "super bacterium", the array of drug-resistant bacteria is becoming stronger.

The development of antibiotic alternatives has become a hot topic in the field of biomedicine. Antimicrobial peptides have become the "star of hope" for antibiotic alternatives as antimicrobial peptides have broad-spectrum and high-efficiency bactericidal activity against bacteria and are not prone to drug resistance. It has been reported that the cell membrane is the main target for antimicrobial peptides, and the aggregation of antimicrobial peptide molecules on the cell membrane results in increased permeability of the cell membrane, causing the cell membrane to lose the barrier function thereof. The development of microbial resistance towards antimicrobial peptides requires substantial changes in the lipid composition of microbial cell membranes.

Antimicrobial peptides are biologically active small molecule polypeptides that are produced by being induced in organisms, the molecular weight thereof being approximately 2000-7000 and the peptide being composed of 20-60 amino acid residues. According to the source, antimicrobial peptides can be divided into: plant antimicrobial peptides, animal antimicrobial peptides, bacterial antimicrobial peptides (also known as bacteriocins, including cationic peptides and neutral peptides, which can be secreted by both Gram-positive and Gram-negative bacteria), human-derived antimicrobial peptides, and the like.

In November 2013, a research group led by Zhang Yun, a researcher at the Kunming Institute of Zoology, Chinese Academy of Sciences, discovered that natural antimicrobial peptides have selective immune activation and regulation functions, and have good preventive and therapeutic effects on sepsis.

In other literature, it has also been reported that human-derived antimicrobial peptides have broad-spectrum antimicrobial effects, and can regulate wound inflammation and promote angiogenesis and epithelial tissue regeneration at wound sites. During wound healing in humans, antimicrobial peptides are one of the key signaling molecules of the endogenous immune system that interact with various cells and various growth factors and coordinate to reach a balanced state, thereby achieving trauma repair.

CN 101111256 A discloses the amino acid sequence of antimicrobial peptide NA_{L} and D-NA_{L}: Ac-Lys-Trp-Lys-Ser-Phe-Leu-Lys-Thr-Phe-Lys-Ser-Ala-Ala-Lys-Thr-Val-Leu-His-Thr-Ala-Leu-Lys-Ala-Ile-Ser-Ser-NH₂. The antimicrobial peptide has antimicrobial activity, a desired level of hemolytic activity, and a broad-spectrum therapeutic index against Gram-positive and Gram-negative bacteria and other microorganisms having cellular or structural components of lipid bilayer membranes.

The major problem of protein drug preparations is poor drug stability. To improve the stability of protein drugs, preparing protein drugs into sterile lyophilized powders in order to enhance the stability of protein drugs could be easily conceived of. For liquid compositions, the stability thereof can be improved by adding an auxiliary material (stabilizer) such as a polyol (e.g., sorbitol, mannitol, glycerol, propylene glycol, etc.), a sugar (e.g., lactose, gum, dextrin, glucose, trehalose, etc.), an amino acid (e.g., glycine, serine, glutamic acid, lysine, etc.), a salt (e.g., citrate, acetate, phosphate, etc.), a surfactant, etc. However, the lyophilized preparation has the defects of complex prescription and process, and therefore it would be of great significance if a stable protein liquid preparation could be provided.

### SUMMARY

The present invention aims to provide an antimicrobial peptide liquid composition in order to solve the growing problem of drug resistance of bacteria and the pain caused by persistent infection in the majority of patients.

The antimicrobial peptide liquid composition provided in the present invention comprises an antimicrobial peptide, at least one stabilizer and a buffer system, the mass concentration of the antimicrobial peptide in the composition being 0.1%o to 10‰; the mass concentration of the stabilizer being 0.5% to 5%, and the buffer system being a phosphate buffer system or an acetate buffer system.

The antimicrobial peptide is a polypeptide of the following general formula:
Section A-I-A-II-Section B; where I is selected from among any of the following amino acid residues: L-leucine, D-leucine, L-valine, D-valine, L-alanine, D-alanine, glycine, L-serine, D-serine, L-lysine, and D-lysine (L- and D-optical isomeric forms of L, A, S, V, and K, and also G);

II is selected from among any of the following amino acid residues: L-leucine, D-leucine, L-valine, D-valine, L-alanine, D-alanine, glycine, L-serine, D-serine, L-lysine, and D-lysine (L- and D-optical isomeric forms of L, A, S, V, and K, and also G);
section A is represented by SEQ ID No: 1, the sequence thereof being: KWKSFLKTFK;
section B is represented by SEQ ID No: 2, the sequence thereof being: KTVLHTALKAISS;
A represents an alanine residue; and
in the general formula, the direction is from the N-terminus to the C-terminus.

Preferably, the antimicrobial peptide is NA_{L} and D-NA_{L}.

The NA_{L} has the sequence of SEQ ID No: 3, the amino acid sequence thereof being: KWKSFLKTFKSAAKTVLHTALKAISS;

The D-NA_{L} has the sequence of SEQ ID No: 4, the amino acid sequence thereof being: KWKSFLKTFKSAAKTVLHTALKAISS (all amino acids except for A at position 13 which has the L-configuration have the D-configuration).

In the name of the antimicrobial peptide, the capital letter D- (not subscript) indicates that, except for a specific site, the antimicrobial peptide is composed of D-configuration amino acids (for example, D-NA_{L} represents that the antimicrobial peptide is completely composed of D-configuration amino acids except for the substitution of L-alanine in the center of a non-polar surface, and the non-polar surface is represented by the capital letter N).

Preferably, the concentration of the antimicrobial peptide is from 0.5‰ to 6‰, more preferably 1‰ to 4‰, and further preferably 1%o to 2‰, and can specifically be 0.2‰, 0.5‰, 1‰, or 2%o.

Preferably, the buffer system is a disodium hydrogen phosphate-citric acid buffer system, wherein the ion concentration of the buffer system in the composition is from 0.01 M to 0.1 M, preferably 0.01 M to 0.02 M, and more preferably 0.015 M.

Preferably, the pH value of the composition is 3.5 to 5.5, and specifically 3.5, 4.5, or 5.5.

The stabilizer described in the present invention can be selected from among at least one of the following: a polyol (e.g., sorbitol, mannitol, glycerol, propylene glycol, etc.), a sugar (e.g., lactose, gum, dextrin, glucose, trehalose, etc.), an amino acid (e.g., glycine, serine, glutamic acid, lysine, etc.), a salt (e.g., citrate, acetate, phosphate, etc.), a surfactant, etc., and not only stabilizes the antimicrobial peptide, but also maintains the bacteriostatic ability of the antimicrobial peptide.

Preferably, the stabilizer is mannitol, wherein the concentration of mannitol in the antimicrobial peptide liquid composition is from 0.5% to 5%, preferably 1% to 2%, and more preferably 1%, and specifically 0.5%, 1%, 1.25%, 1.5%, or 5%.

A stability test shows that the antimicrobial peptide composition provided in the present invention is stable (the condition being 4°C) within at least 24 months.

The antimicrobial peptide composition provided in the present invention is mainly suitable for treating infectious skin diseases. Compared with gel and cream, spray medication can effectively avoid direct contact with skin wounds and acts directly on the lesion, reducing systemic toxic reactions, and has good stability and easy absorption, improving the safety and compliance of clinical medication, and is thus more suitable for clinical needs.

Thus, the preparation form of the composition provided in the present invention is a liquid preparation, and the dosage form thereof can be a spray, solution, gel, emulsion, sol, drop, syrup, suspension, oral liquid, lotion, or liniment, and the like, and is preferably a spray.

The present invention also provides a method for preparing the above antimicrobial peptide liquid composition.

Furthermore, the method for preparing a composition for external use of the present invention is simple, whereby raw and auxiliary materials that are required by each composition are selected, and conventional methods for preparing each dosage form disclosed in the prior art are used.

The method for preparing an antimicrobial peptide liquid composition provided in the present invention comprises the following steps:
1) measuring 70% to 90% of the usage amount of water for injection, separately adding substances that form the buffer system, and stirring until dissolved; then adding a stabilizer to the above solution, and stirring until completely dissolved; and
2) weighing an antimicrobial peptide and adding same to the solution in step 1), stirring until dissolved, and replenishing the water for injection to the full amount so as to obtain an antimicrobial peptide liquid composition.

The method further comprises a step of filtering the above antimicrobial peptide liquid composition by using a 0.22-micron microporous membrane.

According to one embodiment of the present invention, the specific preparation means of a spray preparation is disclosed, in which after the antimicrobial peptide liquid composition is prepared by using the above method, the same is filled into a spray bottle at 5 ml/bottle, and a spray pump is tightened to obtain an antimicrobial peptide spray.

The antimicrobial peptide used in the present invention is a new broad-spectrum anti-infective drug that disrupts bacterial cell membranes in order to achieve the effect of killing bacteria, and has a unique pharmacological function and a strong bactericidal effect, enabling the same to be different from traditional antibiotics and less prone to drug resistance. Meanwhile, the peptide also has substantially no cross-resistance with traditional antibiotics, and thus represents a very effective approach and method to solve the problem of bacterial resistance.

A major problem of protein drug preparations is poor drug stability. To improve the stability of protein drugs, preparing protein drugs into sterile lyophilized powders in order to enhance the stability of protein drugs could be easily conceived of. For liquid compositions, the stability thereof can be improved by adding an auxiliary material (stabilizer) such as a polyol (e.g., sorbitol, mannitol, glycerol, propylene glycol, etc.), a sugar (e.g., lactose, gum, dextrin, glucose, trehalose, etc.), an amino acid (e.g., glycine, serine, glutamic acid, lysine, etc.), a salt (e.g., citrate, acetate, phosphate, etc.), a surfactant, etc. Undoubtedly, the stability of a protein liquid composition is more difficult to guarantee than the stability of a lyophilized powder. The present invention focuses on solving the problem of the stability of an antimicrobial peptide liquid composition.

The research team of the present invention has found that mannitol can significantly increase the stability of antimicrobial peptides compared to other stabilizers such as glycerol, lactose, and glycine.

The research team of the present invention has found that an acidic pH buffer system can enable antimicrobial peptides to be more stable, and buffer systems that can be considered to include a phosphate buffer system (e.g., disodium hydrogen phosphate + citric acid, or disodium hydrogen phosphate + sodium dihydrogen phosphate, etc.), an acetate buffer system (e.g., sodium acetate + phosphoric acid, or sodium acetate + acetic acid, etc.), and a citric acid buffer system (e.g., sodium citrate + acetic acid, or sodium citrate + citric acid, etc.), and with reference to the stability of the antimicrobial peptide and a bacteriostatic test, preferably the phosphate buffer system and the acetate buffer system (comprising disodium hydrogen phosphate, citric acid, sodium acetate, and glacial acetic acid), and more preferably the buffer system composed of disodium hydrogen phosphate and citric acid.

In the composition of the present invention, the ion concentration of the buffer system also has a certain effect on the stability of the antimicrobial peptide, and the inventors discovered through research that the ion concentration of the buffer system is from 0.01 M to 0.1 M, preferably 0.01 M to 0.02 M, and more preferably 0.015 M.

The antimicrobial peptide spray involved in the present invention exhibits good bioavailability and usage safety; can reduce skin and/or wound surface irritation; has high stability, and in long-term coverage of a wound surface, can improve the microenvironment of the wound surface to inhibit the growth of bacteria; enables the rapid repair of wounds; and can promote healing, and shorten the time for a wound to heal, thus having broad prospects in the field of clinical wound care applications.

The antimicrobial peptide composition of the present invention is useful in preparing broad-spectrum anti-infective pharmaceutical preparations for topical and external use; and is also useful in treating local infections, or treating diseases caused by a local infection. The composition is suitable for various primary skin infections caused by pathogenic bacteria including methicillin-resistant Staphylococcus aureus (MRSA), methicillin-sensitive Staphylococcus aureus (MSSA), erythromycin-resistant and sensitive strains of Streptococcus pyogenes, IPM-R and IPM-S strains of Pseudomonas aeruginosa, and especially drug-resistant bacteria, as well as secondary skin infections such as an eczema co-infection and an ulcer co-infection, including persistent infectious diseases such as diabetic foot, burn wound infections, and decubitus ulcer infections, thus having broad application prospects.

The present invention also claims a broad-spectrum anti-infective product for topical and external use, the active ingredients of which comprise the antimicrobial peptide composition provided in the present invention.

By way of example, the product may be a drug or a pharmaceutical preparation.

The present invention also provides a method for locally fighting infection, which comprises the following step: administering to a recipient animal the antimicrobial peptide composition of the present invention so as to fight a local infection.

The present invention also provides a method for treating a disease caused by a local infection, which comprises the following step: administering to a recipient animal the antimicrobial peptide composition of the present invention so as to treat a disease caused by a local infection.

In the present invention, the animal may be a mammal, such as a human; and the animal may also be another infected animal other than a mammal, such as a mouse.

The infection described above is caused by at least one among the following pathogenic bacteria: methicillin-resistant Staphylococcus aureus, methicillin-sensitive Staphylococcus aureus, an erythromycin-resistant strain of Streptococcus pyogenes, an erythromycin-sensitive strain of Streptococcus pyogenes, and IPM-R and IPM-S strains of Pseudomonas aeruginosa; the infection described above comprises at least one among the following: various primary skin infections caused by drug-resistant bacteria, and secondary skin infections such as an eczema co-infection and an ulcer co-infection, including persistent infectious diseases such as diabetic foot, burn wound infections, and decubitus ulcer infections.

### DETAILED DESCRIPTION

The present invention is further described below in conjunction with specific embodiments; however, the present invention is not limited to the following embodiments. The methods mentioned are conventional methods unless otherwise specified, and the raw materials mentioned can be obtained from public commercial sources unless otherwise specified.

The sequence of the antimicrobial peptide (having the sequence of SEQ ID No: 3) used in the following embodiments can be artificially synthesized.

### Embodiment 1. Screening of Stabilizer in Antimicrobial Peptide Composition

Mannitol, glycerol, lactose, and glycine were selected as a stabilizer for an antimicrobial peptide, aqueous solutions of an antimicrobial peptide composition (antimicrobial peptide concentration: 2‰) were separately prepared, and the stability of the antimicrobial peptide composition was investigated, detection indicators including trait, related substances (largest single impurity and total impurities), and content, etc.

Detection method for related substances:
According to a high-performance liquid chromatography test (Chinese Pharmacopoeia 2015 Edition, Volume II, Appendix V D), octadecylsilane-bonded silica gel was used as a filler, and 20 µL of the solution was injected into a liquid chromatograph, and the chromatogram was recorded. Related substances and limits: for known impurities A, B, C, D, and unknown single impurities, each impurity should not exceed 1.0%, and the largest single impurity is summarized by the maximum value of all single impurities; and the total impurities include the sum of impurities A, B, C, D, and other unknown single impurities, and the total impurities should not exceed 3.0%.

In the table below:
antimicrobial peptide (2‰) + mannitol (1%) composition;
antimicrobial peptide (2‰) + glycerol (1%) composition;
antimicrobial peptide (2‰) + lactose (1%) composition; and
antimicrobial peptide (2‰) + glycine (1%) composition.

**Table 1. Investigation Results of Antimicrobial Peptide Compositions (at 25°C)**

| Test Condition (25°C) | | Trait | Related Substance (%) | | Content (%) |
|---|---|---|---|---|---|
| | | | Largest Single Impurity | Total Impurities | |
| 0 days | antimicrobial peptide | colorless clear liquid | 0.19 | 0.32 | 99.87 |
| | antimicrobial peptide + mannitol composition | colorless clear liquid | 0.17 | 0.37 | 100.33 |
| | antimicrobial peptide + glycerol composition | colorless clear liquid | 0.18 | 0.38 | 100.14 |
| | antimicrobial peptide + lactose composition | colorless clear liquid | 0.18 | 0.30 | 99.73 |
| | antimicrobial peptide + glycine composition | colorless clear liquid | 0.18 | 0.31 | 99.99 |
| 1 month | antimicrobial peptide | colorless transparent liquid | 0.25 | 0.53 | 98.51 |
| | antimicrobial peptide + mannitol composition | colorless transparent liquid | 0.17 | 0.43 | 98.94 |
| | antimicrobial peptide + glycerol composition | colorless transparent liquid | 0.20 | 0.49 | 96.52 |
| | antimicrobial peptide + lactose composition | colorless transparent liquid | 0.21 | 0.59 | 96.52 |
| | antimicrobial peptide + glycine composition | colorless transparent liquid | 0.19 | 0.48 | 97.92 |
| 3 months | antimicrobial peptide | colorless transparent liquid | 0.68 | 1.96 | 88.57 |
| | antimicrobial peptide + mannitol composition | colorless transparent liquid | 0.20 | 0.46 | 98.45 |
| | antimicrobial peptide + glycerol composition | colorless transparent liquid | 0.37 | 0.85 | 90.40 |
| | antimicrobial peptide + lactose composition | colorless transparent liquid | 0.39 | 0.96 | 91.02 |
| | antimicrobial peptide + glycine composition | colorless transparent liquid | 0.41 | 0.89 | 93.05 |
| 6 months | antimicrobial peptide | colorless transparent liquid | 0.98 | 3.16 | 78.88 |
| | antimicrobial peptide + mannitol composition | colorless transparent liquid | 0.25 | 0.56 | 97.59 |
| | antimicrobial peptide + glycerol composition | colorless transparent liquid | 0.78 | 1.36 | 83.80 |
| | antimicrobial peptide + lactose composition | colorless transparent liquid | 0.59 | 1.16 | 85.50 |
| | antimicrobial peptide + glycine composition | colorless transparent liquid | 0.61 | 1.26 | 86.89 |

**Table 2. Investigation Results of Antimicrobial Peptide Composition Test (at 4°C)**

| Test Condition (25°C) | | Trait | Related Substance (%) | | Content (%) |
|---|---|---|---|---|---|
| | | | Largest Single Impurity | Total Impurities | |
| 0 days | antimicrobial peptide | colorless clear liquid | 0.19 | 0.32 | 99.87 |
| | antimicrobial peptide + mannitol composition | colorless clear liquid | 0.17 | 0.37 | 100.33 |
| | antimicrobial peptide + glycerol composition | colorless clear liquid | 0.18 | 0.38 | 100.14 |
| | antimicrobial peptide + lactose composition | colorless clear liquid | 0.18 | 0.30 | 99.73 |
| | antimicrobial peptide + glycine composition | colorless clear liquid | 0.18 | 0.31 | 99.99 |
| 6 months | antimicrobial peptide | colorless transparent liquid | 0.27 | 0.58 | 97.51 |
| | antimicrobial peptide + mannitol composition | colorless transparent liquid | 0.18 | 0.41 | 99.24 |
| | antimicrobial peptide + glycerol composition | colorless transparent liquid | 0.23 | 0.53 | 96.42 |
| | antimicrobial peptide + lactose composition | colorless transparent liquid | 0.24 | 0.55 | 96.46 |
| | antimicrobial peptide + glycine composition | colorless transparent liquid | 0.22 | 0.49 | 97.58 |
| 12 months | antimicrobial peptide | colorless transparent liquid | 0.32 | 0.68 | 94.51 |
| | antimicrobial peptide + mannitol composition | colorless transparent liquid | 0.21 | 0.43 | 98.94 |
| | antimicrobial peptide + glycerol composition | colorless transparent liquid | 0.38 | 0.71 | 93.52 |
| | antimicrobial peptide + lactose composition | colorless transparent liquid | 0.35 | 0.68 | 94.52 |
| | antimicrobial peptide + glycine composition | colorless transparent liquid | 0.32 | 0.61 | 95.92 |
| 24 months | antimicrobial peptide | colorless transparent liquid | 0.72 | 2.01 | 85.57 |
| | antimicrobial peptide + mannitol composition | colorless transparent liquid | 0.22 | 0.46 | 98.47 |
| | antimicrobial peptide + glycerol composition | colorless transparent liquid | 0.39 | 0.88 | 90.42 |
| | antimicrobial peptide + lactose composition | colorless transparent liquid | 0.41 | 0.86 | 91.51 |
| | antimicrobial peptide + glycine composition | colorless transparent liquid | 0.42 | 0.87 | 93.44 |
| 36 months | antimicrobial peptide | colorless transparent liquid | 0.96 | 3.02 | 76.55 |
| | antimicrobial peptide + mannitol composition | colorless transparent liquid | 0.23 | 0.52 | 97.86 |
| | antimicrobial peptide + glycerol composition | colorless transparent liquid | 0.79 | 1.16 | 84.81 |
| | antimicrobial peptide + lactose composition | colorless transparent liquid | 0.58 | 1.02 | 85.52 |
| | antimicrobial peptide + glycine composition | colorless transparent liquid | 0.59 | 1.11 | 86.34 |

From the investigation results of the stability of antimicrobial peptide compositions from Tables 1 and 2, it can be seen that mannitol (1%) as the stabilizer of the antimicrobial peptide can ensure that the content of the antimicrobial peptide in the antimicrobial peptide composition solution at 36 months (at 4°C) is still 97% or more.

Considering the stability of different antimicrobial peptide concentrations in different mannitol systems, the stability of different antimicrobial peptide concentrations (2‰ and 0.2‰) in different mannitol (0.5% to 5%) systems was further investigated, and the results are shown in Table 3.

**Table 3. Investigation Results of Stability of Antimicrobial Peptide Compositions in Different Mannitol Systems**

| Test Condition (25°C) | | Trait | Related Substance (%) | | Content (%) |
|---|---|---|---|---|---|
| | | | Largest Single Impurity | Total Impurities | |
| 0 days | antimicrobial peptide | colorless clear liquid | 0.16 | 0.30 | 100.12 |
| | antimicrobial peptide (2‰) + mannitol (0.5%) | colorless clear liquid | 0.15 | 0.29 | 100.23 |
| | antimicrobial peptide (2‰) + mannitol (1%) | colorless clear liquid | 0.17 | 0.30 | 100.18 |
| | antimicrobial peptide (2‰) + mannitol (5%) | colorless clear liquid | 0.18 | 0.31 | 100.31 |
| | antimicrobial peptide (0.2‰) + mannitol (1%) | colorless clear liquid | 0.19 | 0.28 | 99.98 |
| | antimicrobial peptide (0.2‰) + mannitol (1.25%) | colorless clear liquid | 0.18 | 0.27 | 100.11 |
| 3 months | antimicrobial peptide | colorless transparent liquid | 0.26 | 0.55 | 98.42 |
| | antimicrobial peptide (2‰) + mannitol (0.5%) | colorless transparent liquid | 0.22 | 0.38 | 98.94 |
| | antimicrobial peptide (2‰) + mannitol (1%) | colorless transparent liquid | 0.23 | 0.33 | 99.52 |
| | antimicrobial peptide (2‰) + mannitol (5%) | colorless transparent liquid | 0.21 | 0.32 | 98.91 |
| | antimicrobial peptide (0.2‰) + mannitol (1%) | colorless transparent liquid | 0.24 | 0.35 | 98.68 |
| | antimicrobial peptide (0.2‰) + mannitol (1.25%) | colorless transparent liquid | 0.22 | 0.37 | 98.79 |
| 6 months | antimicrobial peptide | colorless transparent liquid | 0.85 | 1.99 | 87.67 |
| | antimicrobial peptide (2‰) + mannitol (0.5%) | colorless transparent liquid | 0.30 | 0.55 | 97.55 |
| | antimicrobial peptide (2‰) + mannitol (1%) | colorless transparent liquid | 0.27 | 0.45 | 99.40 |
| | antimicrobial peptide (2‰) + mannitol (5%) | colorless transparent liquid | 0.29 | 0.46 | 98.82 |
| | antimicrobial peptide (0.2‰) + mannitol (1%) | colorless transparent liquid | 0.26 | 0.43 | 98.62 |
| | antimicrobial peptide (0.2‰) + mannitol (1.25%) | colorless transparent liquid | 0.28 | 0.41 | 98.55 |

It can be seen from Table 3 that when the concentration of an antimicrobial peptide is 2%o and the concentration of mannitol in the composition system is 0.5%, the content of the antimicrobial peptide in the antimicrobial peptide composition solution at 6 months (at 25°C) is 97.55%; and when the concentration of mannitol in the composition system reaches 1% or more, the content of the antimicrobial peptide in the antimicrobial peptide composition solution at 6 months (at 25°C) can be maintained at 98% or more. Therefore, the preferred concentration of mannitol is 1%.

### Embodiment 2. Investigation of Buffer System and Molar Concentration of Buffer System in Antimicrobial Peptide Composition

The buffer system in an antimicrobial peptide composition was investigated according to the combinations in the table below, and the mass concentration of an antimicrobial peptide in each buffer system in the antimicrobial peptide composition in the table below is 4‰.

**Table 4. Buffer System in Antimicrobial Peptide Composition**

| | | |
|---|---|---|
| mixed phosphate buffer system | disodium hydrogen phosphate and citric acid | 0.015 M (pH 4.5) |
| mixed citrate buffer system | sodium citrate and acetic acid | 0.015 M (pH 4.5) |
| mixed acetate buffer system | sodium acetate and phosphoric acid | 0.015 M (pH 4.5) |
| phosphate buffer system | disodium hydrogen phosphate and sodium dihydrogen phosphate | 0.015 M (pH 4.5) |
| acetate buffer system | sodium acetate and acetic acid | 0.015 M (pH 4.5) |
| citrate buffer system | sodium citrate and citric acid | 0.015 M (pH 4.5) |

**Table 5. Investigation Results of Test of Buffer System in Antimicrobial Peptide Composition**

| Test Condition (25°C) | | Trait | Related Substance | | Content (%) |
|---|---|---|---|---|---|
| | | | Largest Single Impurity | Total Impurities | |
| 0 days | disodium hydrogen phosphate and citric acid | colorless clear liquid | 0.16 | 0.26 | 100.1 |
| | sodium citrate and acetic acid | colorless clear liquid | 0.17 | 0.35 | 99.99 |
| | sodium acetate and phosphoric acid | colorless clear liquid | 0.18 | 0.29 | 100.0 |
| | disodium hydrogen phosphate and sodium dihydrogen phosphate | colorless clear liquid | 0.17 | 0.30 | 99.82 |
| | sodium acetate and acetic acid | colorless clear liquid | 0.19 | 0.31 | 99.97 |
| | sodium citrate and citric acid | colorless clear liquid | 0.18 | 0.31 | 99.93 |
| 3 months | disodium hydrogen phosphate and citric acid | colorless transparent liquid | 0.21 | 0.47 | 98.65 |
| | sodium citrate and acetic acid | colorless transparent liquid | 0.29 | 0.73 | 94.91 |
| | sodium acetate and phosphoric acid | colorless transparent liquid | 0.28 | 0.69 | 93.52 |
| | disodium hydrogen phosphate and sodium dihydrogen phosphate | colorless transparent liquid | 0.34 | 0.79 | 92.52 |
| | sodium acetate and acetic acid | colorless transparent liquid | 0.39 | 0.90 | 92.42 |
| | sodium citrate and citric acid | colorless clear liquid | 0.36 | 0.88 | 91.89 |

It can be seen from Table 5 that the choice of the buffer system of disodium hydrogen phosphate and citric acid can ensure that the content of the antimicrobial peptide in the antimicrobial peptide composition solution at 3 months (at 25°C) is still 98% or more. Thus, the buffer system of disodium hydrogen phosphate and citric acid is the preferred buffer system.

For the preferred buffer system of disodium hydrogen phosphate and citric acid, the effect of the ion concentration (0.01 M to 0.1 M) of the buffer system on the antimicrobial peptide stability was investigated, and the investigation results are shown in Table 6.

**Table 6. Effect of Ion Concentration of Buffer System on Antimicrobial Peptide Stability**

| Test Condition (25°C) | | Trait | Related Substance (%) | | Content (%) |
|---|---|---|---|---|---|
| | | | Largest Single Impurity | Total Impurities | |
| 0 days | 0.1M | colorless clear liquid | 0.15 | 0.28 | 100.1 |
| | 0.05M | colorless clear liquid | 0.14 | 0.23 | 100.1 |
| | 0.02M | colorless clear liquid | 0.14 | 0.25 | 100.0 |
| | 0.015M | colorless clear liquid | 0.12 | 0.26 | 101.0 |
| | 0.01M | colorless clear liquid | 0.16 | 0.21 | 100.1 |
| 3 months | 0.1M | colorless transparent liquid | 0.30 | 0.61 | 96.35 |
| | 0.05M | colorless clear liquid | 0.33 | 0.46 | 97.82 |
| | 0.02M | colorless transparent liquid | 0.23 | 0.48 | 97.75 |
| | 0.015M | colorless transparent liquid | 0.21 | 0.48 | 98.21 |
| | 0.01M | colorless clear liquid | 0.42 | 0.68 | 96.57 |

It can be seen from Table 6 that the investigation results of the effect of the ion concentration of the buffer system on the stability of the antimicrobial peptide composition solution show that when the ion concentration is approximately 0.015 M, the content of the antimicrobial peptide in the antimicrobial peptide composition solution at 3 months (at 25°C) can be ensured to still be 98%.

### Embodiment 3. Investigation of pH Value of Antimicrobial Peptide Composition

Mannitol (1%) was selected as a stabilizer and disodium hydrogen phosphate and citric acid was selected as a buffer system (0.015 M), antimicrobial peptide (2‰) composition solutions having different pH values (3.0, 3.5, 4.5, 5.5, 6.5) were formulated, and the stability of the antimicrobial peptide composition under different pH value conditions was investigated, and the results are shown in Table 7.

Meanwhile, hydrochloric acid was selected as a pH regulator, and the pH value of an aqueous antimicrobial peptide solution was adjusted to 4.5; the stability of the antimicrobial peptide was investigated and compared with the antimicrobial peptide composition solution in which disodium hydrogen phosphate and citric acid are used as the buffer system, and the results are shown in Table 8.

**Table 7. Effect of pH Value of Composition on Antimicrobial Peptide Stability**

| Test Condition (25°C)/pH | | Trait | Related Substance (%) | | Content (%) |
|---|---|---|---|---|---|
| | | | Largest Single Impurity | Total Impurities | |
| 0 days | 3.0 | colorless clear liquid | 0.17 | 0.30 | 101.07 |
| | 3.5 | colorless clear liquid | 0.19 | 0.31 | 99.98 |
| | 4.5 | colorless clear liquid | 0.20 | 0.28 | 100.14 |
| | 5.5 | colorless clear liquid | 0.18 | 0.30 | 100.03 |
| | 6.5 | colorless clear liquid | 0.21 | 0.31 | 99.89 |
| 3 months | 3.0 | colorless transparent liquid | 0.29 | 0.53 | 97.51 |
| | 3.5 | colorless transparent liquid | 0.23 | 0.53 | 98.91 |
| | 4.5 | colorless transparent liquid | 0.20 | 0.49 | 98.52 |
| | 5.5 | colorless transparent liquid | 0.22 | 0.51 | 98.59 |
| | 6.5 | colorless transparent liquid | 0.35 | 0.58 | 94.42 |

**Table 8. Effect of Different pH Regulators on Antimicrobial Peptide Stability (pH 4.5)**

| Test Condition (25°C) | | Trait | Related Substance (%) | | Content (%) |
|---|---|---|---|---|---|
| | | | Largest Single Impurity | Total Impurities | |
| 0 days | hydrochloric acid | colorless clear liquid | 0.23 | 0.37 | 100.8 |
| | disodium hydrogen phosphate + citric acid | colorless clear liquid | 0.22 | 0.29 | 101.2 |
| 3 months | hydrochloric acid | colorless transparent liquid | 0.36 | 0.73 | 94.51 |
| | disodium hydrogen phosphate + citric acid | colorless transparent liquid | 0.26 | 0.43 | 98.91 |

It can be seen from Table 7 and Table 8 that when the antimicrobial peptide composition is at a pH value of 3.5 to 5.5, the content of the antimicrobial peptide in the antimicrobial peptide composition solution at 3 months (at 25°C) can be ensured to still be at 98% or more. When the pH value of the aqueous antimicrobial peptide solution was adjusted to 4.5 by using hydrochloric acid, the content of the antimicrobial peptide in the aqueous antimicrobial peptide solution at 3 months (at 25°C) dropped to 95% or less.

### Embodiment 4. Antimicrobial Peptide Spray Composition

### (1) Prescription

### Prescription 1. Prescription composition

| | | |
|---|---|---|
| antimicrobial peptide | 5.00 g | active ingredient |
| mannitol | 50.00 g | stabilizer |
| sodium dihydrogen phosphate | 5.47 g | buffer system |
| citric acid | 6.46 g | buffer system |
| water for injection | add to 5000 ml | solvent |

### Prescription 2. Prescription composition

| | | |
|---|---|---|
| antimicrobial peptide | 5.00 g | active ingredient |
| mannitol | 25.00 g | stabilizer |
| disodium hydrogen phosphate | 6.18 g | buffer system |
| citric acid | 4.13 g | buffer system |
| water for injection | add to 5000 ml | solvent |

### Prescription 3. Prescription composition

| | | |
|---|---|---|
| antimicrobial peptide | 10.00 g | active ingredient |
| mannitol | 25.00 g | stabilizer |
| sodium dihydrogen phosphate | 5.47 g | buffer system |
| citric acid | 6.46 g | buffer system |
| water for injection | add to 5000 ml | solvent |

### Prescription 4. Prescription composition

| | | |
|---|---|---|
| antimicrobial peptide | 2.50 g | active ingredient |
| mannitol | 75.00 g | stabilizer |
| disodium hydrogen phosphate | 3.05 g | buffer system |
| citric acid | 8.36 g | buffer system |
| water for injection | add to 5000 ml | solvent |

### Prescription 5. Prescription composition

| | | |
|---|---|---|
| antimicrobial peptide | 1.00 g | active ingredient |
| mannitol | 50.00 g | stabilizer |
| disodium hydrogen phosphate | 5.47 g | buffer system |
| citric acid | 6.46 g | buffer system |
| water for injection | add to 5000 ml | solvent |

### Prescription 6. Prescription composition

| | | |
|---|---|---|
| antimicrobial peptide | 1.00 g | active ingredient |
| mannitol | 75.00 g | stabilizer |
| disodium hydrogen phosphate | 3.05 g | buffer system |
| citric acid | 8.36 g | buffer system |
| water for injection | add to 5000 ml | solvent |

### (2) Preparation process

1. 70-90% of the prescription amount of water for injection was measured, the prescription amount of disodium hydrogen phosphate and citric acid were separately added, and stirred for 15 minutes until dissolved; then the prescription amount of mannitol was added to the above solution and stirred for 10 minutes until completely dissolved.
2. The prescription amount of the antimicrobial peptide was weighed and added to the above solution, and stirred until dissolved, and the water for injection was replenished to the full amount.
3. The peptide was filtered by using a 0.22-micron microporous membrane, and samples were taken to determine the trait, content, pH value, etc. of the intermediate.

The peptide was filled into a spray bottle at 5 ml/bottle, and a spray pump was tightened to obtain an antimicrobial peptide spray.

After filling, a suitable amount of the finished product was taken for inspection according to the detection methods under the quality standard.

### (3) Quality requirements

1) Related substances: according to a high-performance liquid chromatography test (Chinese Pharmacopoeia 2015 Edition, Volume II, Appendix V D), octadecylsilane-bonded silica gel was used as a filler, and 20 µL of the solution was injected into a liquid chromatograph, and the chromatogram was recorded. Related substances and limits: for known impurities A, B, C, D, and unknown single impurities, each impurity does not exceed 1.0%; the total impurities include the sum of impurities A, B, C, D, and other unknown single impurities, and the total impurities do not exceed 3.0%.
2) pH: should meet the specified pH of 3.5 to 5.5.
3) Content: according to the determination of high-performance liquid chromatography (Chinese Pharmacopoeia 2015 Edition, Volume IV, General Rules 0512), a suitable amount of the product was accurately measured, dissolved with added water and quantitatively diluted into a solution containing 0.25 mg per 1 ml as the test solution; 10 µl was accurately measured and injected into the liquid chromatograph, and the chromatogram was recorded; additionally, a suitable amount of antimicrobial peptide control was taken and subjected to determination by using the same method.

A stability investigation was carried out on Prescription 1 and Prescription 6, and the results are shown in Table 9.

**Table 9. Investigation Results of Antimicrobial Peptide Composition (at 25°C)**

| Prescription | Time | Trait | pH value | Related Substance (%) | | Content (%) |
|---|---|---|---|---|---|---|
| | | | | Largest Single Impurity | Total Impurities | |
| Prescription 1 | 0 months | colorless clear liquid | 4.5 | 0.17 | 0.35 | 100.33 |
| | 1 month | colorless transparent liquid | 4.5 | 0.19 | 0.42 | 98.96 |
| | 3 months | colorless transparent liquid | 4.5 | 0.21 | 0.44 | 98.66 |
| | 6 months | colorless transparent liquid | 4.5 | 0.22 | 0.44 | 98.59 |
| Prescription 6 | 0 months | colorless clear liquid | 4.5 | 0.15 | 0.31 | 100.22 |
| | 1 month | colorless transparent liquid | 4.5 | 0.18 | 0.39 | 99.36 |
| | 3 months | colorless transparent liquid | 4.5 | 0.21 | 0.42 | 98.87 |
| | 6 months | colorless transparent liquid | 4.5 | 0.22 | 0.41 | 98.69 |

As can be seen from Table 9, the content of the antimicrobial peptide in both Prescription 1 and Prescription 6 at 6 months (at 25°C) can be maintained at 98% or more.

### (4) In vitro bacteriostatic and bactericidal tests

The antimicrobial peptide compositions (Prescription 2 and Prescription 4) were tested for in vitro bacteriostatic effect and bactericidal efficacy on clinical isolates of methicillin-resistant Staphylococcus aureus (MRSA), Methicillin-sensitive Staphylococcus aureus (MSSA), Methicillin-resistant coagulase-negative Staphylococcus (MRSCNS), and Methicillin-sensitive coagulase-negative Staphylococcus (MSSCNS). The control drug was vancomycin. The results are shown in Table 10 and 11.

### Test strains:

Standard strain: Staphylococcus aureus ATCC29213 (from the Clinical Laboratory Center of Jiangsu Provincial People's Hospital, and stored by Nanjing Skin Research Institute);

Clinical strains: MRSA, MSSA MRSCNS, and MSSCNS strains were all from the Clinical Laboratory Center of Jiangsu Provincial People's Hospital and stored by Nanjing Skin Research Institute.

**Table 10. In Vitro Bacteriostatic Effect of Antimicrobial Peptide and Vancomycin on Staphylococcus Aureus and Coagulase-negative Staphylococcus**

| Strain | MIC Concentration Range (µg/ml) | | |
|---|---|---|---|
| | Prescription 2 | Prescription 4 | Vancomycin |
| ATCC29213 | 8 | 8 | 1 |
| MRSA | 2 - 16 | 2 - 16 | 0.5 - 4 |
| MSSA | 2 - 16 | 2 - 16 | 0.5 - 4 |
| MRSCNS | 2 - 16 | 2 - 16 | 0.5 - 4 |
| MSSCNS | 2 - 16 | 2 - 16 | 0.5 - 4 |

**Table 11. In Vitro Bactericidal Effect of Antimicrobial Peptide and Vancomycin on Staphylococcus Aureus and Coagulase-negative Staphylococcus**

| Strain | MBC Concentration Range (µg/ml) | | |
|---|---|---|---|
| | Prescription 2 | Prescription 4 | Vancomycin |
| ATCC29213 | 8 | 8 | 4 |
| MRSA | 4 - > 64 | 4 - > 64 | 0.5 - > 16 |
| MSSA | 2 - > 64 | 2 - > 64 | 0.5 - > 16 |
| MRSCNS | 2 - > 64 | 2 - > 64 | 0.5 - > 16 |
| MSSCNS | 2 - > 64 | 2 - > 64 | 0.5 - > 16 |

### (5) In vivo efficacy test

Staphylococcus aureus and Streptococcus pyogenes which are clinically most common in local skin bacterial infections and Pseudomonas aeruginosa which is clinically common in persistent infections were selected as infectious bacteria, and local skin infection models including clinical strains of MRSA (methicillin-resistant Staphylococcus aureus), MSSA (methicillin-sensitive Staphylococcus aureus), erythromycin-resistant and sensitive strains of Streptococcus pyogenes, and IPM-R and IPM-S strains of Pseudomonas aeruginosa were established by means of local infection or mild damage infection of mouse skin^{[1~3]}. By using a 0.5% ofloxacin gel as a control drug, and a vehicle group (1% mannitol, 0.015 M buffer system of disodium hydrogen phosphate and citric acid, pH 4.5) as a negative control group, the efficacy of the antimicrobial peptide spray on a local skin infection caused by the above bacterial strains were quantitatively evaluated. The results are shown in Tables 12 - 14.

The test strains were all from the Clinical Laboratory Center of Jiangsu Provincial People's Hospital and stored by Nanjing Skin Research Institute, and the strain codes were compiled by Nanjing Skin Research Institute.

1‰ (Prescription 1), 0.5‰ (Prescription 4), and 0.2‰ (Prescription 6) of the antimicrobial peptide sprays have a significant inhibitory effect on a local skin infection in mice caused by Staphylococcus aureus (MRSA and MSSA) and Streptococcus pyogenes (erythromycin-sensitive and resistant strains). 1‰ of the antimicrobial peptide spray also has an inhibitory effect on a local skin infection in mice caused by Pseudomonas aeruginosa (IPM-R and IPM-S) (calculation of inhibition rate in antimicrobial peptide group = [1-(concentration of bacteria solution in homogenate of administration group/concentration of bacteria solution in homogenate of vehicle group)] x 100%; calculation of inhibition rate in ofloxacin gel group = [1-(concentration of bacteria solution in homogenate of administration group/concentration of bacteria solution in homogenate of model group)] x 100%). The statistical summary of the bacteriostatic rate after the treatments indicates that the administration amount of antimicrobial peptide is far lower than that of ofloxacin when the same therapeutic effect is achieved.

**Table 12-1. Results of Inhibition Rate Calculation for Skin Lesion Homogenate Culture after Antimicrobial Peptide Spray Treatment of Local Infection of Superficial Skin in Mice Infected with MRSA**

| Strain | Group | Number | Inhibition Rate (%) |
|---|---|---|---|
| SA-R-7 | 1‰ antimicrobial peptide spray (Prescription 1) | 13 | 96 |
| | 0.5‰ antimicrobial peptide spray (Prescription 4) | 13 | 89 |
| | 0.2‰ antimicrobial peptide spray (Prescription 6) | 13 | 84 |
| | 0.5% ofloxacin gel | 13 | 99 |
| | vehicle group (spray solution without antimicrobial peptide) | 13 | / |
| | Model group | 12 | / |
| SA-R-66 | 1‰ antimicrobial peptide spray (Prescription 1) | 13 | 96 |
| | 0.5‰ antimicrobial peptide spray (Prescription 4) | 13 | 82 |
| | 0.2‰ antimicrobial peptide spray (Prescription 6) | 13 | 75 |
| | 0.5% ofloxacin gel | 12 | 81 |
| | vehicle group (spray solution without antimicrobial peptide) | 13 | / |
| | model group | 13 | / |
| SA-R-87 | 1‰ antimicrobial peptide spray (Prescription 1) | 10 | 92 |
| | 0.5‰ antimicrobial peptide spray (Prescription 4) | 10 | 71 |
| | 0.2‰ antimicrobial peptide spray (Prescription 6) | 10 | 53 |
| | 0.5% ofloxacin gel | 10 | 62 |
| | Vehicle group (spray solution without antimicrobial peptide) | 10 | / |
| | model group | 10 | / |
| SA-R-120 | 1‰ antimicrobial peptide spray (Prescription 1) | 13 | 89 |
| | 0.5‰ antimicrobial peptide spray (Prescription 4) | 13 | 45 |
| | 0.2‰ antimicrobial peptide spray (Prescription 6) | 13 | 7 |
| | 0.5% ofloxacin gel | 13 | 79 |
| | vehicle group (spray solution without antimicrobial peptide) | 13 | / |
| | model group | 13 | / |
| SA-R-64 | 1‰ antimicrobial peptide spray (Prescription 1) | 9 | 93 |
| | 0.5‰ antimicrobial peptide spray (Prescription 4) | 10 | 88 |
| | 0.2‰ antimicrobial peptide spray (Prescription 6) | 10 | 81 |
| | 0.5% ofloxacin gel | 10 | 99 |
| | vehicle group (spray solution without antimicrobial peptide) | 10 | / |
| | model group | 10 | / |

**Table 12-2. Results of Inhibition Rate Calculation for Skin Lesion Homogenate Culture after Antimicrobial Peptide Spray Treatment of Local Infection of Superficial Skin in Mice Infected with MSSA**

| Strain | Group | Number | Inhibition Rate (%) |
|---|---|---|---|
| SA-S-24 | 1‰ antimicrobial peptide spray (Prescription 1) | 10 | 99 |
| | 0.5‰ antimicrobial peptide spray (Prescription 4) | 10 | 85 |
| | 0.2‰ antimicrobial peptide spray (Prescription 6) | 10 | 69 |
| | 0.5% ofloxacin gel | 10 | 100 |
| | vehicle group (spray solution without antimicrobial peptide) | 10 | / |
| | model group | 10 | / |
| SA-S-30 | 1‰ antimicrobial peptide spray (Prescription 1) | 10 | 99 |
| | 0.5‰ antimicrobial peptide spray (Prescription 4) | 10 | 90 |
| | 0.2‰ antimicrobial peptide spray (Prescription 6) | 10 | 81 |
| | 0.5% ofloxacin gel | 10 | 100 |
| | vehicle group (spray solution without antimicrobial peptide) | 10 | / |
| | model group | 10 | / |
| SA-S-66 | 1‰ antimicrobial peptide spray (Prescription 1) | 11 | 100 |
| | 0.5‰ antimicrobial peptide spray (Prescription 4) | 11 | 99 |
| | 0.2‰ antimicrobial peptide spray (Prescription 6) | 11 | 96 |
| | 0.5% ofloxacin gel | 11 | 99 |
| | vehicle group (spray solution without antimicrobial peptide) | 11 | / |
| | model group | 11 | / |

**Table 13. Results of Inhibition Rate Calculation for Skin Lesion Homogenate Culture after Antimicrobial Peptide Spray Treatment of Infection of Local Skin in Mice with Streptococcus pyogenes**

| Strain | Group | Number | Inhibition Rate (%) |
|---|---|---|---|
| SH-S-15 erythromycin-sensitive | 1‰ antimicrobial peptide spray (Prescription 1) | 10 | 100 |
| | 0.5‰ antimicrobial peptide spray (Prescription 4) | 10 | 96 |
| | 0.2‰ antimicrobial peptide spray (Prescription 6) | 10 | 93 |
| | 0.5% ofloxacin gel | 10 | 100 |
| | vehicle group (spray solution without antimicrobial peptide) | 10 | / |
| | model group | 10 | / |
| SH-S-8 erythromycin-sensitive | 1‰ antimicrobial peptide spray (Prescription 1) | 10 | 86 |
| | 0.5‰ antimicrobial peptide spray (Prescription 4) | 10 | 75 |
| | 0.2‰ antimicrobial peptide spray (Prescription 6) | 10 | 61 |
| | 0.5% ofloxacin gel | 10 | 100 |
| | vehicle group (spray solution without antimicrobial peptide) | 10 | / |
| | model group | 10 | / |
| SH-R-14 erythromycin-resistant | 1‰ antimicrobial peptide spray (Prescription 1) | 10 | 99 |
| | 0.5‰ antimicrobial peptide spray (Prescription 4) | 10 | 94 |
| | 0.2‰ antimicrobial peptide spray (Prescription 6) | 10 | 92 |
| | 0.5% ofloxacin gel | 10 | 100 |
| | vehicle group (spray solution without antimicrobial peptide) | 10 | / |
| | model group | 10 | / |
| SH-R-15 erythromycin-resistant | 1‰ antimicrobial peptide spray (Prescription 1) | 10 | 86 |
| | 0.5‰ antimicrobial peptide spray (Prescription 4) | 10 | 82 |
| | 0.2‰ antimicrobial peptide spray (Prescription 6) | 10 | 80 |
| | 0.5% ofloxacin gel | 10 | 100 |
| | vehicle group (spray solution without antimicrobial peptide) | 10 | / |
| | model group | 10 | / |
| SH-R-17 erythromycin-resistant | 1‰ antimicrobial peptide spray (Prescription 1) | 10 | 84 |
| | 0.5‰ antimicrobial peptide spray (Prescription 4) | 10 | 59 |
| | 0.2‰ antimicrobial peptide spray (Prescription 6) | 10 | 41 |
| | 0.5% ofloxacin gel | 10 | 100 |
| | vehicle group (spray solution without antimicrobial peptide) | 10 | / |
| | model group | 10 | / |

**Table 14-1. Results of Inhibition Rate Calculation for Skin Lesion Homogenate Culture after Antimicrobial Peptide Spray Treatment of Infection of Local Skin in Mice with IPM-R**

| Strain | Group | Number | Inhibition Rate (%) |
|---|---|---|---|
| PA-R-85 | 1‰ antimicrobial peptide spray (Prescription 1) | 10 | 64 |
| | 0.5‰ antimicrobial peptide spray (Prescription 4) | 10 | 31 |
| | 0.2‰ antimicrobial peptide spray (Prescription 6) | 10 | 5 |
| | 0.5% ofloxacin gel | 10 | 100 |
| | vehicle group (spray solution without antimicrobial peptide) | 10 | / |
| | model group | 10 | / |
| PA-R-50 | 1‰ antimicrobial peptide spray (Prescription 1) | 10 | 27 |
| | 0.5‰ antimicrobial peptide spray (Prescription 4) | 10 | 20 |
| | 0.2‰ antimicrobial peptide spray (Prescription 6) | 10 | 13 |
| | 0.5% ofloxacin gel | 9 | 100 |
| | vehicle group (spray solution without antimicrobial peptide) | 10 | / |
| | model group | 10 | / |
| PA-R-15 | 1‰ antimicrobial peptide spray (Prescription 1) | 10 | 34 |
| | 0.5‰ antimicrobial peptide spray (Prescription 4) | 10 | 15 |
| | 0.2‰ antimicrobial peptide spray (Prescription 6) | 10 | 1 |
| | 0.5% ofloxacin gel | 10 | 95 |
| | vehicle group (spray solution without antimicrobial peptide) | 10 | / |
| | model group | 10 | / |
| | 1‰ antimicrobial peptide spray (Prescription 1) | 10 | 61 |
| | 0.5‰ antimicrobial peptide spray (Prescription 4) | 10 | 47 |
| | 0.2‰ antimicrobial peptide spray (Prescription 6) | 10 | 7 |
| | 0.5% ofloxacin gel | 10 | 100 |
| | vehicle group (spray solution without antimicrobial peptide) | 10 | / |
| | model group | 10 | / |
| | 1‰ antimicrobial peptide spray (Prescription 1) | 10 | 43 |
| | 0.5‰ antimicrobial peptide spray (Prescription 4) | 10 | 40 |
| | 0.2‰ antimicrobial peptide spray (Prescription 6) | 10 | 38 |
| | 0.5% ofloxacin gel | 10 | 100 |
| | vehicle group (spray solution without antimicrobial peptide) | 10 | / |
| | model group | 10 | / |

**Table 14-2. Results of Inhibition Rate Calculation for Skin Lesion Homogenate Culture after Antimicrobial Peptide Spray Treatment of Infection of Local Skin in Mice with IPM-S**

| Strain | Group | Number | Inhibition Rate (%) |
|---|---|---|---|
| PA-S-28 | 1‰ antimicrobial peptide spray (Prescription 1) | 10 | 35 |
| | 0.5‰ antimicrobial peptide spray (Prescription 4) | 10 | 24 |
| | 0.2‰ antimicrobial peptide spray (Prescription 6) | 10 | 20 |
| | 0.5% ofloxacin gel | 10 | 100 |
| | vehicle group (spray solution without antimicrobial peptide) | 10 | / |
| | model group | 10 | / |
| PA-S-39 | 1‰ antimicrobial peptide spray (Prescription 1) | 10 | 66 |
| | 0.5‰ antimicrobial peptide spray (Prescription 4) | 10 | 40 |
| | 0.2‰ antimicrobial peptide spray (Prescription 6) | 10 | 18 |
| | 0.5% ofloxacin gel | 10 | 100 |
| | Vehicle group (spray solution without antimicrobial peptide) | 10 | / |
| | model group | 10 | / |
| PA-S-41 | 1‰ antimicrobial peptide spray (Prescription 1) | 10 | 40 |
| | 0.5‰ antimicrobial peptide spray (Prescription 4) | 10 | 29 |
| | 0.2‰ antimicrobial peptide spray (Prescription 6) | 10 | 12 |
| | 0.5% ofloxacin gel | 10 | 100 |
| | vehicle group (spray solution without antimicrobial peptide) | 10 | / |
| | model group | 10 | / |

### References:

1. Guidelines for Clinical Research of Antimicrobial Drugs for Simple and Complicated Skin Infections, issued by the US FDA in July 1998, and translated under the organization of the Center for Drug Evaluation in 2009
2. Xu Shuyun et al., Pharmacological Experimental Methodology, People's Medical Publishing House, November 2006, Third Edition
3. Compilation of guiding principles for preclinical research of new drugs (Western drugs), Pharmaceutical Administration Bureau of the Ministry of Health of the People's Republic of China, 1993.7

### Industrial Application

The antimicrobial peptide composition of the present invention is useful in preparing broad-spectrum anti-infective pharmaceutical preparations for topical and external use, and is suitable for various primary skin infections caused by pathogenic bacteria including methicillin-resistant Staphylococcus aureus (MRSA), methicillin-sensitive Staphylococcus aureus (MSSA), erythromycin-resistant and sensitive strains of Streptococcus pyogenes, IPM-R and IPM-S strains of Pseudomonas aeruginosa, and especially drug-resistant bacteria, as well as secondary skin infections such as an eczema co-infection and an ulcer co-infection, including persistent infectious diseases such as diabetic foot, burn wound infections, and decubitus ulcer infections, thus having broad application prospects.

## Claims

1. An antimicrobial peptide liquid composition, comprising an antimicrobial peptide, at least one stabilizer, and a buffer system, the mass concentration of the antimicrobial peptide in the composition being 0.1%o to 10%o, and the buffer system being a phosphate buffer system or an acetate buffer system;
the antimicrobial peptide is a polypeptide of the following general formula:
Section A-I-A-II-Section B;
where I is selected from among any of the following amino acid residues: L-leucine, D-leucine, L-valine, D-valine, L-alanine, D-alanine, glycine, L-serine, D-serine, L-lysine, and D-lysine;
II is selected from among any of the following amino acid residues: L-leucine, D-leucine, L-valine, D-valine, L-alanine, D-alanine, glycine, L-serine, D-serine, L-lysine, and D-lysine;
section A is represented by SEQ ID No: 1, the sequence thereof being: KWKSFLKTFK;
section B is represented by SEQ ID No: 2, the sequence thereof being: KTVLHTALKAISS;
A represents an alanine residue; and
in the general formula, the direction is from the N-terminus to the C-terminus.

2. The composition according to claim 1, wherein the mass concentration of the antimicrobial peptide is 0.5‰ to 6‰, preferably 1‰ to 4‰, and more preferably 1‰ to 2‰.

3. The composition according to claim 1 or 2, wherein the buffer system is a disodium hydrogen phosphate-citric acid buffer system, wherein the ion concentration of the buffer system in the composition is from 0.01 M to 0.1 M, preferably 0.01 M to 0.02 M, and more preferably 0.015 M.

4. The composition according to any one of claims 1-3, wherein the antimicrobial peptide is NA_{L} or D-NA_{L};
where the NA_{L} has the sequence of SEQ ID No: 3, the amino acid sequence thereof being: KWKSFLKTFKSAAKTVLHTALKAISS;
and the D-NA_{L} has the sequence of SEQ ID No: 4, the amino acid sequence thereof being: KWKSFLKTFKSAAKTVLHTALKAISS; and all amino acids except for A at position 13 have the D-configuration.

5. The composition according to any one of claims 1-4, wherein the stabilizer is selected from among at least one of the following: a polyol, an amino acid, a salt, and/or a surfactant;
preferably, the stabilizer is mannitol, wherein the mass concentration of mannitol in the composition is from 0.5% to 5%, preferably 1% to 2%, and more preferably 1%.

6. The composition according to any one of claims 1-5, wherein the composition has a pH value of 3.5 to 5.5.

7. The composition according to any one of claims 1-6, wherein the preparation form of the composition is a liquid preparation, and the dosage form thereof is a spray, solution, gel or emulsion, sol, drop, syrup, suspension, oral liquid, lotion, or liniment, preferably a spray.

8. An application of the composition of any one of claims 1-7 in preparing a broad-spectrum anti-infective product for topical and external use.

9. An application of the composition of any one of claims 1-7 in the treatment of a local infection and/or in the treatment of a disease caused by a local infection.

10. The application according to claim 8 or 9, wherein the product is a drug product;
the infection is caused by at least one among the following pathogenic bacteria: methicillin-resistant Staphylococcus aureus, methicillin-sensitive Staphylococcus aureus, erythromycin-resistant strains of Streptococcus pyogenes, an erythromycin-sensitive strain of Streptococcus pyogenes, and IPM-R and IPM-S strains of Pseudomonas aeruginosa; and
the infection comprises at least one among the following: various primary skin infections caused by drug-resistant bacteria, and secondary skin infections such as an eczema co-infection and an ulcer co-infection, including persistent infectious diseases such as diabetic foot, burn wound infections, and decubitus ulcer infections.

11. A broad-spectrum anti-infective product for topical and external use, the active ingredients of which comprising the composition of any one of claims 1-7.

12. The product according to claim 11, wherein the product is a drug product;
the infection is caused by at least one among the following pathogenic bacteria: methicillin-resistant Staphylococcus aureus, methicillin-sensitive Staphylococcus aureus, an erythromycin-sensitive strain of Streptococcus pyogenes, a Streptococcus pyogenes erythromycin-sensitive strain, and IPM-R and IPM-S strains of Pseudomonas aeruginosa; and
the infection comprises at least one among the following: various primary skin infections caused by drug-resistant bacteria, and secondary skin infections such as an eczema co-infection and an ulcer co-infection, including persistent infectious diseases such as diabetic foot, burn wound infections, and decubitus ulcer infections.

13. A method for locally fighting infection, comprising the following step: administering to a recipient animal the composition of any one of claims 1-7 or the product of claim 11 or 12 so as to fight a local infection.

14. A method for treating a disease caused by a local infection, comprising the following step: administering to a recipient animal the composition of any one of claims 1-7 or the product of claim 11 or 12 so as to treat a disease caused by a local infection.
